Europäisches Patentamt

European Patent Office

Office européen des brevets

(11) Publication number: **0 116 526**
**B1**

# (12) EUROPEAN PATENT SPECIFICATION

(45) Date of publication of patent specification: **08.02.89**

(21) Application number: **84850052.6**

(22) Date of filing: **14.02.84**

⑩⑩ Consolidated with 84900906.3/0169836 (European application No./publication No.) by decision dated 07.03.88.

(51) Int. Cl.⁴: **A 61 M 25/02**

(54) A device for fixation of catheters and the like.

(30) Priority: **15.02.83 SE 8300795**

(43) Date of publication of application:
**22.08.84 Bulletin 84/34**

(45) Publication of the grant of the patent:
**08.02.89 Bulletin 89/06**

(84) Designated Contracting States:
**AT BE CH DE FR GB IT LI NL SE**

(56) References cited:
**DE-C- 458 201**
**US-A-3 825 010**
**US-A-4 217 902**

(73) Proprietor: **Gustavsson, Bengt**
**Bergsbogatan 29**
**S-421 79 Västra Frölunda (SE)**
(73) Proprietor: **Curelaru, Ioan**
**Dr. Lindsgatan 3**
**S-413 25 Göteborg (SE)**
(73) Proprietor: **Linder, Lars-Erik**
**Valebergsvägen 319**
**S-427 00 Billdal (SE)**

(72) Inventor: **Gustavsson, Bengt**
**Bergsbogatan 29**
**S-421 79 Västra Frölunda (SE)**
Inventor: **Curelaru, Ioan**
**Dr. Lindsgatan 3**
**S-413 25 Göteborg (SE)**
Inventor: **Linder, Lars-Erik**
**Valebergsvägen 319**
**S-427 00 Billdal (SE)**

(74) Representative: **Roth, Ernst Adolf Michael et al**
**GÖTEBORGS PATENTBYRA AB Box 5005**
**S-402 21 Göteborg (SE)**

Courier Press, Leamington Spa, England.

## Description

Technical field

The present invention refers to a device for fixation of a tube, like a drainage tube or a catheter, to the skin and comprising holding means for the tube or for a connection member for it and at least one pointed fixing member intended to penetrate the skin for fixing the device.

Background of the invention

Catheters are usually relatively small, resilient and flexible tubes made of natural or synthetic rubber or of various polymers and they extend from outside a patient's body into and through his tissues and cavity organs for the purpose of introducing medicines, nutritive solutions, contrast liquids etc., for taking samples and for several other purposes.

The success of catheterization and often the life of the patient is determined to a large degree of proper placement and fixation of the catheter in a correct position. Accidental dislodgement of the catheter is frequently encountered in children, disoriented or combative adults and in patients who might frequently be turned about in bed or moved to different departments of the hospital. The problem becomes even more important for patients, who need catheters for the whole of their life course, such as those with intestinal insufficiency and parenteral nutrition at home.

If the fixation of the catheter is unsatisfactory it may be inserted further into the patients body, partially or entirely withdrawn or perform to and fro movements. For example in the case of central venous catheters the frequency of inadvertant catheter dislodgement is about 25%. Among the complications caused by catheter displacement can be mentioned injuries on the catheterized and neighboring organs, which in some cases can be so serious that the patient dies; chronic irritation of the organ walls, which for central venous catheters may result in thrombosis or fatal subacute endocarditis; the catheter kinks or twists at which its function is stopped or deteriorioated; possible readings of intracavitary pressures become false; skin irritations and risk of infections through the catheter insertion site.

Among the most common methods which today are used for fixation of the catheter to the skin the following can be mentioned:

1. Fixation by adhesive tapes, which however in may cases gives an unsatisfactory fixation. Sweating and possible growth of hair causes the tape to pull off at which the catheter can slip out.

2. Skin sutures, which today is the safest method of catheter fixation. The disadvantages with this method is however that it can cause inflammations and scar formation. Besides it is less adequate for soft catheters and for long term use (more than a month), when the sutures become loose and are gradually rejected by the patients organisms.

3. Surgical methods, which however are complicated and time consuming and have to be performed by specialists.

In the US—A—4.164.943 a device for catheter fixation is disclosed, which comprises a disc with a number of needles, which when the disc is rotated penetrates the skin and is fixed thereto. Holding means for the catheter is arranged on the disc. This device does however not provide a safe fixation, as the needles easily are displaced out of their position at sudden movements. Besides it is difficult to choose and to observe the position for the introduction of needles.

In the DE—C—458.201 it is disclosed a device for attaching a drainage tube to the skin by means of a safety-pin. This device does however not prevent to and fro movements of the tube.

In the US—A—4.217.902 it is disclosed a hemostatic clip having ridges, eylets or apertures for cooperating with a pair of pliers, by means of which the clip is applied to the wound flaps. The clip is not provided with any holding means for a catheter or the like.

Purpose and most important features of the invention

The purpose of the present invention is to provide a device of the kind mentioned in the introduction, which provides a simple, quick and very safe fixation of a tube like a catheter. It should further permit a fixation during a theoretically unlimited duration of time without the need to be changed. A condition for this is that irritation and infection are avoided, which demands a very strong fixation. This has according to a first alternative of the invention, been achieved by the fact that said fixing member comprises a surgical clip or a pincer having jaws provided with teeth or needles. According to a second alternative of the invention this has been achieved by a device of the kind mentioned in the introduction, wherein said holding means is arranged on a plate intended to be applied to the skin, and particularly by the fact that said fixing member during the application of the device is movable with respect to the holding means and connected or connectable to said holding means and that said fixing means comprises at least one needle or the like intended to be passed through openings in the plate and that locking means are provided for fixing said fixing member to said holding means

Description of the drawings

The invention will now be further described with reference to some embodiments shown in the accompanying drawings.

Fig. 1 shows in perspective and on an enlarged scale a first embodiment of the invention.

Fig. 2 shows a somewhat modified embodiment of the device according to Fig. 1.

Fig. 3 shows in a front view another embodiment of the device in the applied position.

Fig. 4 shows a device according to Fig. 3 in the applied position.

Fig. 5a shows schematically a further embodiment of the device according to the invention fixed to, for example, an arm of a patient.

Fig. 5b shows in a small view a device according to Fig. 5a.

Fig. 5c is a vertical section through the device.

Fig. 6a—d show a further embodiment of the device, at which

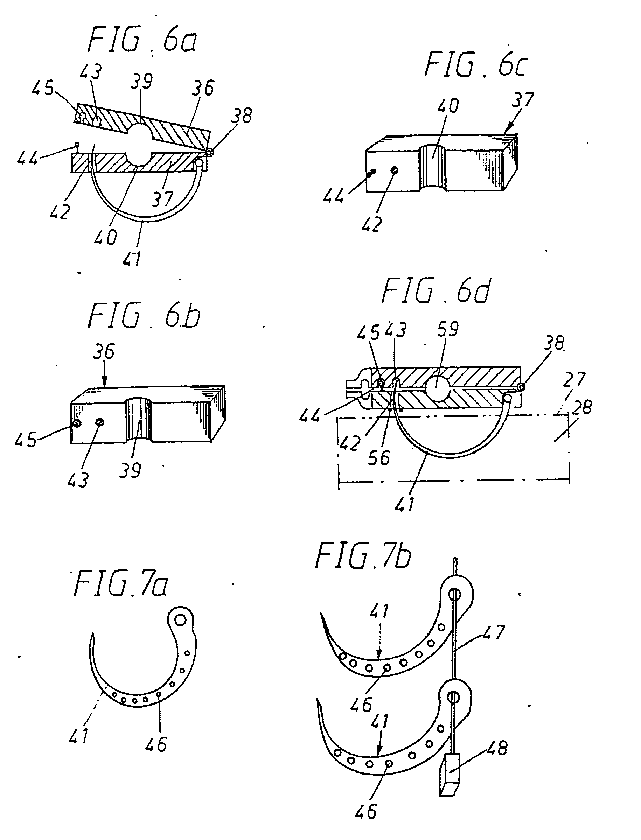

Fig. 6a shows a vertical section through the device,

Fig. 6d and 6c show in perspective the upper and lower plate resp. of the device.

Fig. 6d shows the device applied to the skin of the patient.

Fig. 7a and 7b show a pair of special designs of the fixation needles of the device.

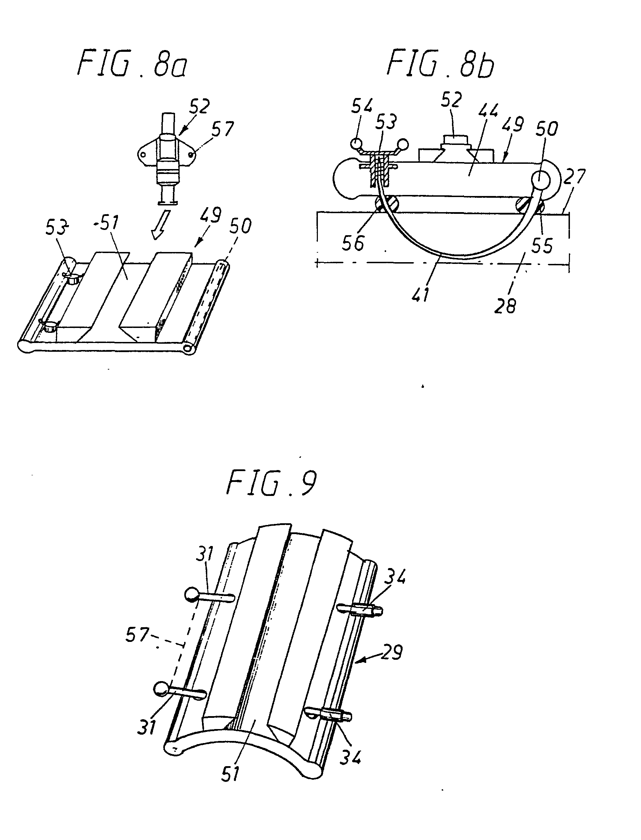

Fig. 8a—b show another embodiment of the invention especially designed for fixation of the connection member of a catheter, at which

Fig. 8a shows the device in perspective.

Fig. 8b shows a vertical section through the device fixed to the skin of the patient,

Fig. 9 shows a modified embodiment of the device according to Fig. 8.

Description of the embodiments

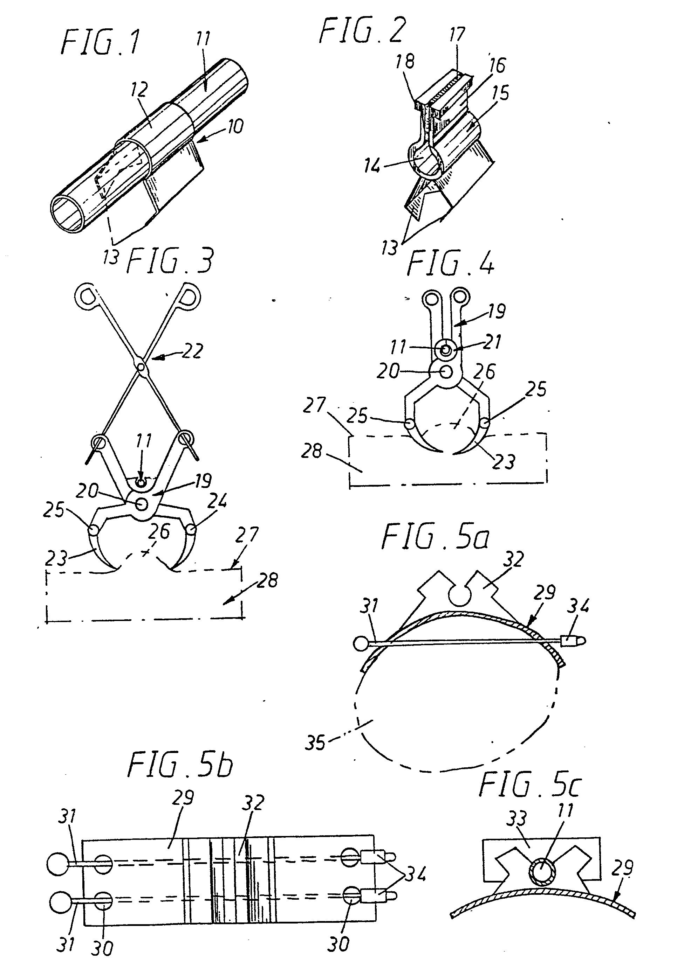

In its simplest embodiment the device according to the invention consists of a surgical clip 10 (Fig. 1), which may be made of a maleable metal. e.g. silver, which is non-irritating for the tissues. The clip 10 is designed with a channel 12 intended to receive a catheter 11, in which channel the catheter is retained in clamped position. This type of fixation device can be suitable for catheters having a small outer diameter (1—3 mm) and for only short duration of catheterization (about 7—10 days).

The insertion is made in the following manner:

The catheter is passed between the jaws 13 of the clip and into the channel 12. A skin fold is made by means of one or two surgical pincers, whereupon the teeth of the jaws 13 are placed at a base of the skin fold. The jaws of the clip are closed by means of an anathomical pincer or by a pair of pincers specially constructed and used for application and removal of surgical clips. During this last step the channel 12 is closed, whereby the catheter 11 is locked therein at the same time as the clip is attached to the skin. The removal of the clip is brought about in the same manner as for conventional surgical clips.

In Fig. 2 is shown a modified embodiment of the clip according to Fig. 1 whereby the clip at the base of the jaws 13 remote from its teeth is provided with a channel 14, which is formed by a slotted cylinder 15 made e.g. from thin elastic steel. Two rectangular plates 16 are arranged to extend upwards from the edges of the slot, and these plates delimit a space 17 between them. The upper edges of the plates 16 are made thicker and they have recesses 18 for the application of a surgical pincer.

The channel 14 which is delimited by the cylinder 15 has a diameter which is somewhat less than the outer diameter of the catheter which shall be locked therein. The inner side of the cylinder can be rough or coated with a thin layer of a polymer having a high coefficient of friction (e.g. polyuretane) in order to prevent the catheter from sliding to and fro in the channel 14.

The application is made in the following manner. The plates 16 are pulled apart by means of a small surgical pincer (see e.g. Fig. 3) and the catheter is placed in the cylinder 15. The pincer is removed and the slotted cylinder forms the channel 14 when the plates 16 moves back to their initial positions, which is brought about by the elasticity by the cylinder itself. The application is then performed in the same manner as for the first mentioned clip.

The advantages with the clip according to Fig. 2 compared to that according to Fig. 1 is that the risk for damage of the catheter from the teeth of the clip is eliminated.

In Figs. 3 and 4, there is shown a device resembling a crab pincer and which also is based on the principle for surgical clips. The device can be designed with one or two pincers. The pincer 19 is preferably made of stainless steel (medical grade). Immediately above the pivot axis 20 of the pincer between its arms and rigidly connected to these a resilient cylinder 28 is arranged, which is slotted at its upper side. The inner side of the cylinder can be rough or coated with a thin layer of an elastic polymer; having a high friction coefficient (e.g. polyuretane). the jaws, of the pincer are thin curved surgical needles 23 of e.g. titanium, which is an inert material to human tissues.

At the application of the device the needles 23 penetrate the skin of the patient and into the subcutaneous tissues. The penetration of the needles 23 is limited by small rounded projections 25 which at the bottom form the end of the arms of the pincer. The channel which is formed by the cylinder 21 has an internal diameter which is somewhat smaller than the outer diameter of the catheter 11 which is to be fixed thereby.

The device is applied in the following manner. The arms of the pincer 19 are moved apart by means of a fine surgical pincer 22 (Fig. 3), whereby the cylinder 21 is opened and the catheter 11 is placed therein. A skin fold 26 is made by means of two or several surgical pincers, whereupon the jaws of the pincers are located at the base of the skinfold. The surgical pincer 22 is removed with caution, whereupon the flexible cylinder 21 resumes its initial position and closes the channel. At the same time the needles 23 penetrate the skin 27 and the subcutaneous tissues 28 of the patient. The penetration is limited by the projections 25.

The device is removed in the corresponding manner by the arms of the pincers being pushed apart by means of a surgical pincer.

The device is suited for all types of catheters and for almost unlimited duration of catheterization.

The device according to the embodiment shown in Figs. 5a—c consists of a curved plate 29 of a hard plastic material or by stainless steel (medical grade) provided with two or four orifices 30 through which one or two needles preferably from non-alloy titanium can be inserted. On top of the plate 29 a catheter holder 32 is fitted which is fixedly connected thereto. The catheter holder 32 is provided with a lock 33, which together with the catheter holder form a channel for the catheter 11.

The device is applied in the following manner. A skin fold is made by means of two surgical pliers. The curved plate 29 is applied to the fold between the surgical pincer, the needles 31 are inserted through the orifices 31 and the skin and subcutaneous tissue of the patient and are fixed by means of tips 34 which are screwed thereon firmly and ensure a secure fixation of the entire device and prevent the needles 31 from sliding to and fro in the tissues of the patient.

The device is particularly intended for curved surfaces, e.g. on an arm 35, but when made in small dimensions it can be adapted to other positions for the catheterization. It is convenient for all duration of catheterization.

The device shown in Figs. 6a—d consists of two plates e.g. of stainless steel (medical grade), an upper plate 36 and a lower plate 37, which are interconnected by means of a pivot 38. The plates 36, 37 are provided with grooves 39 and 40 respectively which are situated just in front of each other and which when the plates are brought together form a cylindrical channel 59 for a catheter. A curved surgical needle 41 is pivotably connected to the lower plate 37 and the lower plate 37 has a through passage 42 for the needle 41 and the upper plate 36 a recess arranged in front of said passage for receiving the tip of the needle. A member which delimits the movement of the needle may be arranged in the passage 42, e.g. spring projections. At the entrance to said passage 42 there can also be arranged such projections 56, which possibly can be designed as a clamping member, which can be clamped about the needle 41 and prevent movement of this. One of the plates 37 has a press button 44, which cooperates with a recess 45 in the other plate 36 for locking the two plates to each other.

The device can be provided with one or two curved surgical needles 41, which should be made of titanium which is an inert material to human tissues. The needles can at their bases be connected to the pivot shaft which interconnects the two plates 36, 37. If however only one needle is used, such as shown in Figs. 6a—d, this can be hingedly connected to the lower plate 37. The needle or needles 41 respectively can have perforations 46 such as shown in Figs. 7a and b whereby it can be penetrated by fibres of connective tissue. A biological fixation of the needles in the tissue of the patient is thereby secured and movement of the needle and transport of bacteria from the skin and into the tunnel formed by the needle are avoided.

The tip of the needle may be conventional, whereby in order to bring about a safe fixation the recess 43 should be filled with a material having a high friction coefficient, and be sufficiently soft to permit penetration of the needle (e.g. rubber or polyuretane). The tip of the needle can have the shape of an arrow tip, which gives a very secure fixtion but which causes problems when the needle is removed. It can be shaped as a javelin tip, whereby the above problem is reduced. It can furthermore be threaded, such as shown e.g. in Fig. 8b and it can be fixed by means of a protected cap provided with internal threads. Other fixation devices are not necessary. It can further be provided with small barbs, which engage with recesses e.g. in a protective cap.

In Fig. 7b there are shown two needles 41 mounted on a common shaft 47 on which also the plates 36, 37 can be mounted. The shaft 47 can be prolonged with a handle 48, which allows insertion of the needles without aid of particular equipment (e.g. Hagerdon's needle driver). This is preferred particularly in emergency situations when such special equipment is not available.

The device according to Figs. 6a—d is applied in the following manner.

A skin fold is made by means of two surgical pincers. The needle 41 or the needles are inserted at the base of the skin fold by means of a Hagerdon's needle driver or by means of the handle 48 if the device is provided with such a handle. The upper plate 36 can thereupon be moved upwards and the catheter is placed in the recess 40 of the lower plate 37. The surgical pincer can now be removed and the upper plate 36 can be moved down and fixation take place by means of the press button 44 or by means of a threaded protective cap if the needle tip is threaded.

The device shown in Figs. 8a—b consists of a single plate 49 of a hard plastic material (e.g. polystyrene or Teflon) or of stainless steel. The plate 49 is provided with a shaft 50 for supporting two curved surgical needles 41.

The tips of the needles are threaded and are moved upwards through the passages in the plate 49, where they are fixed by means of a couple of protective caps 53. These are preferably provided with a handle 54 for making the turning easier. The projection 55 on the needle 41 near its base delimits the penetration of the needle into the tissues. The projections 56 shown at the entrance to the needle passages of the plate 49 are intended to stablize the device. They can possibly be designed as clamping members, which can be clamped about the needles 41 for fixing them securely.

The plate at its upper side has a fixing device formed as a dovetailed groove 51 in which a connection member 52 for a catheter can be inserted and fixed. The fixation of the connection member 52 to the groove 51 can also be made via a connection piece (not shown) which is designed for different sizes of the connection members intended for different catheter sizes.

The application technique is similar to that for the device according to Figs. 6a—d. After the plate 49 has been fixed the connection member 52 is inserted into the groove 51 and its wings 57 are retained only by friction from the walls of the groove. The foremost and rearmost ends of the connection member 52 are situated outside the edges of the plate 49 and allow connection of infusion, or drainage systems and manipulation of the catheter.

The device shown in Fig. 9 is also intended for fixation of the connection member 52 of the catheter. The device comprises a curved plate 29 which is fixed to the skin by means of two needles 31 in a manner corresponding to that of the device according to Figs. 5a—c. The tips of the needles 31 can be threaded and be fixed by means of a couple of screw caps 34. The plate 29 is at its upper side provided with a device forming a dovetailed groove 51 in order to receive a connection member 52 of a catheter. The needles 31 can be connected to a shaft 57 extending between them in order to facilitate their insertion.

The invention is of course not limited to the embodiments shown but can be varied within the scope of the claims. In the text, only catheters have been described. But it is to be understood that the device also is suitable for fixation of drainage tubes, probes and tubes of different types.

## Claims

1. A device for the fixation of a tube for supplying or removing a substance to or from the body, like a catheter or a drainage tube, to the skin and comprising holding means (12; 15; 21) for said member (11) or for a connection member (52) for it, and at least one pointed fixing member (13, 23), that is intended to penetrate the skin for fixing the device, characterized in, that said fixing member is connected or connectable to said holding means (12; 15; 27) and comprises a surgical clip (10) or a pincer (19) having jaws provided with teeth (13) or needles (23).

2. A device for fixation of a tube for supplying or removing a substance to or from the body, like a catheter or a drainage tube, to the skin and comprising holding means (32; 36; 37; 49, 51) for said member (11) or for a connection member (52) for it, said holding means being arranged on a plate (29, 36, 37, 49) intended to be applied to the skin, and at least one pointed fixing member (31; 41) intended to penetrate the skin for fixing the device, characterized in, that said fixing member during the application of the device is movable with respect to the holding means and that said fixing means (31, 41) comprises at least one needle (31, 41) intended to be passed through openings (30) in the plate (29) and that locking means (34, 56) are provided for fixing said fixing member to said holding means.

3. A device according to claim 2, characterized in, that said locking means comprises a cap (34) attachable over the point of the needle (31).

4. A device according to claim 2, characterized in, that the fixing member comprises at least one curved needle (41) rotatably connected to the plate (36, 37, 49), the point of which is intended to be passed through an opening (42) and be locked thereto.

5. A device according to claim 4, characterized in that in or in connection to said opening there are arranged friction increasing means (56).

6. A device according to claim 4 or 5, characterized in that said plate comprises a pair of hingedly connected parts, an upper (36) and a lower (37) part, which are provided with a recess each (39, 40) which when the parts are brought together form a passage (59) for receiving the tube and that the parts are lockable to each other in brought together position.

7. A device according to any of claims 3—6, characterized in, that the point of the needle (31, 41) is threaded and lockable to said plate (29, 36, 37, 49) by means of a screw member (34).

8. A device according to any of claims 3—6, characterized in that the point of the needle (31, 41) is provided with barbs or the like.

9. A device according to any of the claims 3—8, characterized in that at least two needles (31, 41) are used, which are arranged on a common axle (47, 57) for simultaneous manipulation of the needles.

10. A device according to any of the claims 3—9, characterized in that the needle (31, 41) is perforated (46).

## Patentansprüche

1. Vorrichtung zur Fixierung eines Schlauches für die Zufuhr oder Abfuhr einer Substanz in einen bzw. aus einem Körper, beispielsweise eines Katheters oder eines Drainageschlauches, an der Haut, umfassend Haltemittel (12; 15; 21) für den Schlauch (11) oder für ein diesen haltendes Befestigungsorgan (52) sowie wenigstens ein spitz auslaufendes Fixierorgan (13; 23) zum Eindringen in die Haut und zur Befestigung der Vorrichtung, dadurch gekennzeichnet, daß das Fixierorgan mit den Haltemitteln (12; 15, 21) verbunden oder verbindbar ist und eine chirurgische Klammer (10) oder Zange (19) enthält, die Backen mit Zähnen (13) oder Nadeln (23) aufweist.

2. Vorrichtung zur Fixierung eines Schlauches für die Zufuhr oder Abfuhr einer Substanz in einen bzw. aus einem Körper, beispielsweise eines Katheters oder eines Drainageschlauches, an der Haut, umfassend Haltemittel (32; 36; 37; 49; 51) für den Schlauch (11) oder für ein diesen haltendes Befestigungsorgan (52), die an einer an der Haut anbringbaren Platte (29, 36, 37, 49) angeordnet sind, sowie wenigstens ein spitz auslaufendes Fixierorgan (31; 41) zum Eindringen in die Haut und zur Befestigung der Vorrichtung, dadurch gekennzeichnet, daß das Fixierorgan (31, 41) während der Befestigung der Vorrichtung relativ zu den Haltemitteln beweglich ist und wenigstens eine durch Öffnungen (30) in der Platte (29) steckbare Nadel (31, 41) enthält und daß Verriegelungsmittel (34, 56) zur Festlegung des Fixierorgans an den Haltemitteln vorgesehen sind.

3. Vorrichtung nach Anspruch 2, dadurch gekennzeichnet, daß die Verriegelungsmittel eine Kappe (34) aufweisen, die auf die Spitze der Nadel (31) aufsteckbar ist.

4. Vorrichtung nach Anspruch 2, dadurch gekennzeichnet, daß das Fixierorgan wenigstens eine gebogene Nadel (31) enthält, die schwenkbar mit der Platte (36, 37, 49) verbunden ist und deren Spitze durch eine Öffnung (42) steckbar und darin fixierbar ist.

5. Vorrichtung nach Anspruch 4, dadurch gekennzeichnet, daß in der Öffnung (42) oder in Verbindung mit der Öffnung reibungserhöhende Mittel (56) vorgesehen sind.

6. Vorrichtung nach einem der Ansprüche 4 oder 5, dadurch gekennzeichnet, daß die Platte aus einem Paar schwenkbar miteinander verbundener Teile, nämlich einem oberen (36) und einem unteren Teil (37) besteht, von denen jedes eine Nut (39, 40) aufweist, die im zusammengesetzten Zustand der beiden Teile miteinander verriegelbar sind und einen Kanal (59) zur Aufnahme des Schlauches bilden.

7. Vorrichtung nach einem der Ansprüche 3 bis 6, dadurch gekennzeichnet, daß die Spitze der Nadel (31, 41) ein Gewinde aufweist und mittels eines Schraubgliedes (34) an der Platte (29, 36, 37, 49) festlegbar ist.

8. Vorrichtung nach einem der Ansprüche 3 bis 6, dadurch gekennzeichnet, daß die Spitze der Nadel (31, 41) Widerhaken o. dgl. aufweist.

9. Vorrichtung nach einem der Ansprüche 3 bis 8, dadurch gekennzeichnet, daß wenigstens zwei Nadeln (31, 41) vorgesehen sind, die auf einer gemeinsamen Achse (47, 57) für ihre gleichzeitige Betätigung sitzen.

10. Vorrichtung nach einem der Ansprüche 3 bis 9, dadurch gekennzeichnet, daß die Nadel (31, 41) gelocht (46) ist.

**Revendications**

1. Dispositif pour la fixation sur la peau d'un tube pour introduire une substance dans le corps ou pour retirer une substance du corps, comme un cathéter ou un tube de drainage, et comprenant des moyens de retenue (12; 15; 21), pour ledit élément (11) ou pour un élément de liaison (52) pour ce dernier, et au moins un élément de fixation pointu (13; 23), qui est destiné à pénétrer dans la peau pour la fixation du dispositif, caractérisé par le fait que ledit élément de fixation est relié ou apte à être relié auxdits éléments de retenue (12; 15, 27) et comprend un étrier de serrage chirurgical (10) ou une pince (19) présentant des mâchoires dotées de dents (13) ou d'aiguilles (23).

2. Dispositif pour la fixation à la peau d'un tube pour introduire une substance dans le corps ou pour retirer une substance du corps, comme un cathéter ou un tube de drainage, et comprenant des moyens de retenue (32; 36; 37; 49) pour ledit élément (11) ou pour un élément de liaison (52) pour ce dernier, lesdits moyens de retenue étant disposés sur une plaque (29, 36, 37, 49) destinée à être appliquée sur la peau, et au moins un élément de fixation pointu (31; 41) destiné à pénétrer dans la peau pour la fixation du dispositif, caractérisé par le fait que ledit élément de fixation pendant l'application du dispositif est déplaçable par rapport aux moyens de retenue, et que ledit moyen de fixation (31, 41) comprend au moins une aiguille (31, 41) destinée à traverser des ouvertures (30) de la plaque (29), et que des moyens de verrouillage (34, 56) sont prévus pour fixer ledit élément de fixation auxdits moyens de retenue.

3. Dispositif selon la revendication 2, caractérisé par le fait que ledit moyen de verrouillage comprend un capuchon (34) susceptible d'être fixé sur la pointe de l'aiguille (31).

4. Dispositif selon la revendication 2, caractérisé par le fait que l'élément de fixation comprend au moins une aiguille courbe (41), liée de façon tournante à la plaque (36, 37, 49), dont la pointe est destinée à traverser une ouverture (42) et à y être verrouillée.

5. Dispositif selon la revendication 4 caractérisé par le fait que dans, ou en liaison avec, ladite ouverture, sont disposés des moyens (56) pour augmenter le frottement.

6. Dispositif selon la revendication 4 ou 5, caractérisé par le fait que ladite plaque comprend deux parties reliées de façon articulée, à savoir une partie supérieure (36) et une partie inférieure (37), qui sont dotées chacune d'une cavité (39, 40), lesquelles, lorsque les parties sont rassemblées, forment un passage (59) destiné à recevoir le tube, et que les parties sont verrouillables l'une à l'autre dans leur position assemblée.

7. Dispositif selon l'une des revendications 3—6, caractérisé par le fait que la pointe de l'aiguille (31, 41) est filetée et verrouillable sur ladite plaque (29, 36, 37, 49) au moyen d'un élément à vis (34).

8. Dispositif selon l'une des revendications 3—6, caractérisé par le fait que la pointe de l'aiguille (31, 41) est dotée de barbes ou similaires.

9. Dispositif selon l'une des revendications 3—8, caractérisé par le fait qu'au moins deux aiguilles (31, 41) sont utilisées, lesquelles sont disposées sur un axe commun (47, 57), en vue d'une manipulation simultanée des aiguilles.

10. Dispositif selon l'une des revendications 3—9, caractérisé par le fait que l'aiguille (31, 41) est perforée (46).

FIG.1

FIG.2

FIG.3

FIG.4

FIG.5a

FIG.5b

FIG.5c

FIG . 6a

FIG . 6c

FIG . 6b

FIG . 6d

FIG . 7a

FIG . 7b

# FIG.8a

# FIG.8b

# FIG.9